Europäisches Patentamt

(19) European Patent Office

Office européen des brevets

(11) Numéro de publication: **0 182 696**
**B1**

(12) FASCICULE DE BREVET EUROPEEN

(45) Date de publication du fascicule du brevet: **23.01.91**

(51) Int. Cl.⁵: **A 61 K 31/71, A 61 K 9/14**

(21) Numéro de dépôt: **85402107.8**

(22) Date de dépôt: **31.10.85**

(54) **Médicament insoluble administré localement dans l'oreille.**

(30) Priorité: **14.11.84 FR 8417397**

(43) Date de publication de la demande:
**28.05.86 Bulletin 86/22**

(45) Mention de la délivrance du brevet:
**23.01.91 Bulletin 91/04**

(84) Etats contractants désignés:
**AT BE CH DE GB IT LI LU NL SE**

(56) Documents cités:
**FR-A-2 542 616**
**US-A-4 025 620**

(73) Titulaire: **Grimberg, Georges Serge**
**123 rue de l'Université**
**F-75007 Paris (FR)**
(73) Titulaire: **Martin, Henri André**
**44 bld des Belges**
**F-69006 Lyon (FR)**
(73) Titulaire: **Mallein, René**
**10, rue Bournes**
**F-69004 Lyon (FR)**

(72) Inventeur: **Grimberg, Georges Serge**
**123 rue de l'Université**
**F-75007 Paris (FR)**
Inventeur: **Martin, Henri André**
**44 bld des Belges**
**F-69006 Lyon (FR)**
Inventeur: **Carraz, Maurice**
**décédé**
**(FR)**
Inventeur: **Mallein, René**
**10, rue Bournes**
**F-69004 Lyon (FR)**

(74) Mandataire: **Madeuf, Claude Alexandre Jean**
**CABINET MADEUF 3, avenue Bugeaud**
**F-75116 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## EP 0 182 696 B1

**Description**

Toutes les gouttes auriculaires utilisées à ce jour sont des solutions parfaites de différentes substances complètement solubles.

De ce fait, elles n'ont qu'une action limitée dans le temps car elles ne permanent pas, elles s'écoulent après avoir plus ou moins été en contact avec les muqueuses malades.

L'objet de la présent invention est une composition topique auriculaire à base d'antibiotique(s) seul(s) ou associé(s) à des anti-inflammatoires, d'une substance insoluble qui tapisse la muqueuse et qui forme un dépôt surcelle-ci, caractérisée en ce que la substance insoluble est la nystatine. La substance insoluble est administrée localement dans l'oreille afin d'y former un dépôt réparti sur la muqueuse qui agira par son activité physique soit comme support diluant de principes actifs en poudre, soit d'éponge et pourra aussi agir par son activité thérapeutique propre.

A cette substance insoluble, seront donc associés des antiseptiques et/ou des antibiotiques et/ou des anti-inflammatoires.

Le volume en poudre obtenu par la substance insoluble et les autres substances sera sensiblement le même que le volume obtenu en rajoutant une certaine quantité de sérum physiologique. De cette manière, la dilution solide ou liquide des substances telles que des antiseptiques et/ou des antibiotiques et/ou des anti-inflammatoires sera la même.

La substance insoluble devra être bien supportée par l'orielle.

La substance insoluble qui répond à ces différentes obligations et permet l'obtention de cette "poudre-goutte auriculaire" est la nystatine.

Elle permet de maintenir en place soit des principes actifs dilués en poudre, soit une solution de principes actifs.

Il est bien évident que l'on peut choisir une autre poudre et utiliser en plus des antibiotiques, un antifongique soluble.

Conformément à l'invention, le médicament insoluble est administré localement dans l'oreille.

Diverses autres caractéristiques de l'invention ressortent d'ailleurs de la description détaillié qui suit.

On donne ci-après, à titre d'exemples, les compositions des médicaments insolubles administrés localement dans l'oreille.

### Example No. 1

Antibiotique q.s.p. activité thérapeutique
Anti-inflammatoires q.s.p. activité thérapeutique
Nystatine en poudre fine q.s.p. 10 ml.

### Exemple No. 2

Antibiotique q.s.p. activité thérapeutique
Anti-inflammatoires q.s.p. activité thérapeutique
Nystatine en poudre q.s.p. 10 ml.
Sérum physiologique 10 ml.

Etude sur animal

La toxicité et la pharmacologie n'ont rien montré d'anormal et donnent les résultats escomptés, à savoir: le médicament formé par la nystatine, un antibiotique et un anti-inflammatoire n'est pas toxique.

Etude sur humain

L'étude à été faite en double aveugle et comporte la comparaison de six produits sur 120 malades ayant une otite chronique.

| Produits No. | 1 | 2 | 3 | 4 | 5 | 6 |
|---|---|---|---|---|---|---|
| Antibiotiques | + | + | + | + | + | + |
| Anti-Inflammatoire | O | + | O | + | O | + |
| Nystatine | O | O | + | + | + | + |
| Serum Physiologique | + | + | + | + | O | O |

Viennent en tête, les produits 4 et 6 qui donnent les meilleurs résultats.

Les produits 3 et 5 sont comparables entre eux et donnent des résultats supérieures aux produits 1 et 2.

2

## EP 0 182 696 B1

**Revendications**

1. Composition topique auriculaire à base d'antibiotique(s) seul(s) ou associé(s) à des anti-inflammatoires, d'une substance insoluble qui tapisse la muqueuse et qui forme un dépôt sur celle-ci, caractérisée en ce que la substance insoluble est la nystatine.

2. Composition selon la revendication 1, caractérisée en ce que les produits solubles sont des antimicrobiens seuls ou associés à des anti-inflammatoires.

3. Composition selon la revendication 1 ou 2, caractérisé en ce que le volume V final du produit en poudre correspond à une concentration des produits solubles dans la poudre, comparable à celle de ces mêmes produits solubles, obtenue quand on rajoutera un volume V de sérum physiologique au produit en poudre.

**Patentansprüche**

1. Zusammensetzung zur lokalen Anwendung im Ohr auf Basis eines Antibiotikums bzw. von Antibiotika allein oder in Verbindung mit entzündungshemmenden Mitteln, in Form einer unlöslichen Substanz, welche die Schleimhaut bedeckt und auf dieser ein Depot bildet, dadurch gekennzeichnet, daß die unlösliche Substanz Nystatin ist.

2. Zusammensetzung gemäß Anspruch 1, dadurch gekennzeichnet, daß die löslichen Mittel antimikrobielle Mittel entweder allein oder in Verbindung mit entzündungshemmenden Mitteln sind.

3. Zusammensetzung gemäß Anspruch 1 oder 2, dadurch gekennzeichnet, daß das Endvolumen V der puderförmigen Substanz einer Konzentration der löslichen Mittel im Puder entspricht, die mit jener Konzentration der gleichen Mittel in löslicher Form vergleichbar ist, die man erhält, wenn ein Volumen V eines physiologischen Serums der puderförmigen Substanz hinzugefügt wird.

**Claims**

1. Ear topic composition with a base of antibiotic substance(s) alone or associated with anti-inflammatory substances, of an insoluble substance which lines the mucous membrane and which forms a deposit thereupon, characterized in that the insoluble substance is nystatin.

2. Composition according to claim 1, characterized in that the soluble products are antimicrobian substances, alone or associated with anti-inflammatory substances.

3. Composition according to claim 1 or 2, characterized in that the final volume V of powder product corresponds to a concentration of the soluble products in the powder, which can be compared to that of these same soluble products obtained when there will be added a volume V of physiological solution to the powder product.

3